# EUROPEAN PATENT SPECIFICATION

(11) **EP 1 282 813 B1**
(45) Date of publication and mention of the grant of the patent: **21.06.2006**
(21) Application number: 00956443.6
(22) Date of filing: 04.08.2000
(51) Int. Cl.: G01N 33/48, G01N 33/50

(54) **USE OF BLUE COPPER PROTEINS**
VERWENDUNG VON BLAUEN KUPFERPROTEINEN
UTILISATION DE PROTÉINES DE CUIVRE BLEUES

(30) Priority: 06.08.1999 US 147339 P
(43) Date of publication of application: 12.02.2003
(73) Proprietor: Tibotec BVBA, 2800 Mechelen (BE)
(72) Inventor: DIERYNCK, Inge, B-2610 Wilrijk (BE); ROELANT, Christiaan, Hubert, Simon, B-3000 Leuven (BE); PAUWELS, Rudi, Wilfried, Jan, B-2820 Bonheiden (BE); VAN ACKER, Koenraad, Lodewijk, August, B-9140 Temse (BE); DE KERPEL, Jan, Octaaf, Antoon, B-9340 Lede (BE)
(74) Representative: Daelemans, Frank F.R.
(86) International application number: PCT/EP2000/007895
(87) International publication number: WO 2001/011358

(56) References cited:
- WO-A-97/09431
- US-A- 5 210 019
- HOITINK, C.W.G. ET AL.: "Isolation and sequencing of the Alcaligenes denitrificans azurin-encoding gene: comparison with the genes encoding blue copper proteins from Pseudomonas aeruginosa and Alcaligenes faecalis." GENE, vol. 90, 1990, page 15-20 XP002150598
- 'The Copper Proteins', [Online] Retrieved from the Internet: <URL:http://web.archive.org/web/20030815081 102/http://www.chm.bris.ac.uk/motm/caerulop lasmin/copper_proteins/>

### SEQUENCE LISTING

<110> TIBOTEC NV
<120> USE OF COLORED METAL BINDING PROTEINS
<130> TIP-4-PCT
<140>
   <141>
<150> US 60/147.339
   <151> 1999-08-06
<160> 3
<170> Patent In Ver. 2.1
<210> 1
   <211> 35
   <212> DNA
   <213> Artificial Sequence
<220>
   <223> Description of Artificial Sequence:5' primer BCP-1C
<400> 1
   gacagaagct tcgccaccat gctggcaaaa gccac 35
<210> 2
   <211> 30
   <212> DNA
   <213> Artificial Sequence
<220>
   <223> Description of Artificial Sequence:3' primer BCP-1NC
<400> 2
   gacagaattc agttgctcag cttgagcgtg 30
<210> 3
   <211> 98
   <212> DNA
   <213> Artificial Sequence
<220>
   <223> Description of Artificial Sequence: 5' primer BCP-2C
<400> 3
   gacagaagct tcgccaccat ggagacagac acactcctgc tatgggtact gctgctctgg 60
   gttccaggtt ccactggtga catgctggca aaagccac                         98

## Description

The present invention is directed to the field of reporter molecules and tags that may be used to monitor a target substance in a biological system. More particularly, the present invention relates to the use of an apo metal binding protein as a reporter molecule or tag. An apo metal binding protein may be bound to a protein or tissue or introduced into a cell. The invention also relates to methods of utilizing the apo metal binding protein for detecting a cell expressing a protein of interest, localizing a protein in a cell, and designing a therapeutic agent for treating a disease or infection.

### Background

Several methods exist to monitor a target substance in a biological system, including monitoring activities inside a cell and intracellular processes. These methods utilize various reporter molecules and genes and other molecular tags and include, for example, the formation of fusion proteins with coding sequences for chloramphenicol acetyl transferase (CAT), gluceronidase (GUC), beta-galactosidase (bGAL) and luciferases (LUC). Many of these reporters and tags may be used as indicators of processes that would be hard to detect otherwise. Silhavy, T. J. & Beckwith, J. R. Microbiol. Rev. (49), 398 (1985); Gould, S. J. & Subramani, S. Anal. Biochem. (175), 5 (1988); and Stewart, G. S. A. B. & Williams, P. J. Gen. Microbiol. (138), 1289 (1992). Many uses of these reporter genes have been described extensively in the prior art. There use, however, has been limited because they require extra manipulations. For example, the fixing of cell preparations or the addition of exogenous substrates or cofactors makes it difficult to incorporate the use of these reporters in highly automated assays. In contrast, the use of another reporter gene, the green fluorescent protein (GFP), is not limited by these restrictions but suffers from other disadvantages, such as background noise and the need for complex and expensive equipment for detection. Cormack, B. P., et al. Gene, 173(1):33-38 (1996); Kroes, S. J., et al. Eur. J. Biochem. 240(2), 342-351 (1996).

Furthermore, until recently, detection in automated assays was based on enzymatic or fluorimetric methods for reasons of sensitivity. As detection at the microscopic level has developed, however, it has become technically possible to use markers without amplification of the marker itself, or signal amplification. Thus, the development of novel reporter genes or markers that have none of the above limitations and disadvantages are desired.

### Summary of the Invention

To achieve these and other advantages, and in accordance with the purpose of the invention as embodied and broadly described herein, the present invention, in one aspect, provides a method of monitoring a target substance in a biological system comprising providing a biological system with a target substance that is labeled with a blue copper protein. Conditions are provided which permit the blue copper protein to emit a signal and the signal is observed or measured. The target substance is monitored based on the signal. The method of monitoring may comprise determining the location of the target substance and/or quantifying the amount of target substance in the biological system.

In another embodiment, the invention, using the method of monitoring a target substance described above, provides for a method of determining the cytotoxicity of a drug of interest. The target substance, a cell, is labeled with a blue copper protein and is exposed to the drug of interest. The cell is monitored and the cytotoxicity of the drug of interest is determined by whether the cell is influenced by the drug of interest.

The invention also provides for a method of labeling a protein of interest comprising fusing a protein of interest to a blue copper protein. In a further embodiment, the invention provides a method of labeling a cell of interest comprising introducing into the cell a blue copper a blue copper protein or by binding to the membrane of the cell a blue copper protein.

The invention further provides a method for detecting a cell expressing a protein of interest comprising introducing into the cell a DNA molecule having a DNA sequence encoding the protein of interest and an additional DNA molecule having a DNA sequence encoding a blue copper protein. Conditions are then provided which permit expression of the blue copper protein and the protein of interest. Conditions are also provided which permit the blue copper protein to emit a signal. The cell expressing the protein of interest is detected by observing or measuring the signal of the blue copper protein.

In another embodiment, the invention provides a method for localizing a protein of interest in a cell comprising introducing into the cell a DNA molecule comprising a DNA sequence encoding the protein of interest, linked to an additional DNA sequence encoding a blue copper protein. The two sequences are linked such that the protein produced by the DNA molecule will have the protein of interest fused to the blue copper protein. Conditions are provided which permit expression of the fused protein. Conditions are also provided which permit the blue copper protein to emit a signal. The location of the fused protein, and thereby the location of the protein of interest in the cell, is determined by observing or measuring the signal.

The invention also provides a method of screening a therapeutic agent for treating a disease. A cell that is a target of the disease is labeled with the blue copper protein and conditions are provided which permit the protein to emit a signal. Whether the therapeutic agent is effective treating the disease is determined by monitoring the signal observed or measured from the cell. In a further embodiment, the methods of the invention may be used to screen a therapeutic agent for treating a virus or bacterial infection comprising labeling the virus or the bacteria with a blue copper protein. Conditions are provided which permit the blue copper protein to emit a signal and the signal is observed or measured. The effectiveness of the therapeutic agent for treating the virus or bacterial infection is determined by observing or measuring the signal from the virus or the bacteria.

The invention also contemplates a eucaryotic cell comprising a DNA sequence that encodes a blue copper protein. In one embodiment, the eucaryotic cell is an animal cell, the blue copper protein is azurin and the DNA sequence comprises the azu gene.

Additional objects and advantages of the invention will be set forth in part in the description that follows, and in part will be apparent from the description, or may be learned by practice of the invention. The objects and advantages of the invention will be realized and attained by means of the elements and combinations particularly pointed out in the appended claims.
It is to be understood that both the foregoing general description and the following detailed description are exemplary and explanatory only and are not restrictive of the invention as claimed.

### Brief Description Of The Drawings

**Figure 1** depicts the primers used to amplify the *azu* gene from bacterial DNA by PCR: the 5' primers BCP-1C or BCP-2C, and the 3' primer BCP-1NC.
**Figure 2** is a map of the pLNCX-BCP 1 vector: 5' MoMuLVLTR: 145-733; Ψ⁺ (extended packaging signal): 803-1612; Neomycin resistance gene (Neo^{r}): 1656-2450; Immediate early CMV promoter (Pcmv): 2800-3617; BCP1-gene: 3622-4701; 3' MoMuLV LTR: 4128-4720; Ampicillin resistance gene (B-lactamase, Amp^{r}): 6023-6865. The pLNCX-BCP1 was deposited at the Belgian Co-ordinated Collections of MicroOrganisms located at the Universiteit Gent- Laboratorium voor Moleculaire Biologie on August 3, 2000, and the accession number is LMBP4156.
**Figure 3** A-D depict the western blots of cell lysates. The blots confirm the expression of azurin.

### Detailed Description Of The Invention

Reference will now be made in detail to the presently preferred embodiments of the invention. The invention, in one aspect, provides a method of monitoring at least one target substance in a biological system comprising labeling a target substance in the biological system with a blue copper protein. Conditions are then provided which allow the blue copper protein to emit a signal and the signal may then be observed or measured. The signal may be used to monitor the target substance including, but not limited to, the location or position of the target substance within the biological system and/or the quantity of the target substance in the biological system.

An apo metal binding protein, as define herein, is any protein or peptide that acquires a detectable color upon the binding of one or more metal ions or changes in color upon binding of a metal ion or an additional metal ion. The color obtained or the change in color observed may also vary with the oxidation state of the metal ion. In one embodiment, the invention utilizes this characteristic as a marker for the analysis of a biological process including, but not limited to, any process inside or outside cells, in *in vitro* assays or to observe cells or processes in tissues or in whole organisms. In a further embodiment, the apo metal binding protein includes groups or families of metal binding proteins that carry an intense color or color change upon binding with a metal.

Apo metal binding proteins acquire a detectable color or change in color upon binding of a metal ion as opposed to proteins that generate a color by an enzymatic reaction or proteins that contain a luminophore. Thus, apo metal binding proteins, which acquire an intrinsic, easily detectable color, provide a novel marker.

Apo metal binding proteins are a heterogeneous collection of proteins from various sources including blue copper proteins in bacteria and mammalian proteins like haemoglobulin, catalase and transferrin. The major advantage of the use of apo metal binding proteins as markers compared to the use of existing reporter genes or markers is the fact that they can be easily detected without the need for extra manipulations or the use of expensive and sophisticated equipment. As many of these proteins do not occur in mammalian cells they are perfect markers to introduce into such cells (e.g. in diagnostic assays).

To date, apo metal binding proteins have not been used as markers in analytical testing, diagnostic testing or screening for drugs. One patent (U.S. Patent No. 5,210,019) describes the use of azurin, a blue copper protein, to detect pseudomonas bacteria. However these bacteria naturally express azurin, i.e., the protein was not introduced into the bacteria with the intention of using it as a marker of an internal or external process. Azurin is only used to detect the presence of the bacteria as such. Similarly, PCT application, WO 99/30730A1, discloses the use of the metal binding protease superoxide dismutase, however, there is no disclosure of the metal binding protein as a reporter molecule or marker.

The apo metal binding protein of the present invention is a blue copper protein, a derivative or mutant of a blue copper protein or any protein or peptide sequence with the copper binding characteristics of a blue copper protein sequence as described below. In living cells, copper carries out several physiological functions in association with specific metabolic proteins, e.g. participation in electron transfer reactions, various enzymatic reactions and transport or storage of the metal itself. Copper proteins and more specifically, blue copper proteins, consist of a wide variety of proteins (families) normally with a color that can be changed into other colors depending on pH and/or mutations. Kroes, S. J., et al., Eur. J. Biochem. 240(2), 342-351 (1996) and E. Solomon, Chemical Reviews; 1996; 96(7); 2239-2314. Copper proteins may be classified according to their biological function, by copper centre type, by number of prosthetic centres and by sequence similarity.

Alternative to copper proteins, other apo metal proteins may include proteins linked to a transition metal like Co, Ni, Mn, Zn, Fe, or an alkali metal like Ca, Mg, Na and K.

In the present invention the apo metal binding protein is at least one blue copper protein, or a derivative or mutant of a blue copper protein. According to the classification of copper proteins by their copper centre, the blue copper proteins form a distinct class. In this class, they can be divided into three groups: the small blue copper proteins (e.g. azurins, pseudo-azurins, plastocyanin family, and phytocyanin family), the blue oxidases (e.g. ascorbate oxidase, ceruloplasmin, and laccase) and nitrite reductase.

Blue copper proteins, also referred to as "type-1" copper proteins, include but are not limited to small proteins that bind a single copper atom and which are characterized by their very intense absorption in the region of 600 nm due to a charge transfer transition from a cysteine residue. Not to be limited as to theory, there is a relation between the EPR spectrum of the blue copper proteins, the relative intensity of the important peaks in the UV/VIS absorption spectrum, and the structure of the active site. Based on these observed parameters, the most widely observed blue copper proteins may be divided into four structural classes.

The first class consists of the classical blue copper proteins, with its most studied example plastocyanin. Here the cysteine and the two histidine residues form a plane with the Cu(II) atom lying slightly above. The S_{Cys}-Cu(II) distance is very short (± 2.1 Å). Axial to this plane, the fourth residue methionine, is at a large distance (± 2.9 Å). The overall structure of the active site is distorted tetrahedral or trigonal (C₃ᵥ).

A second group of blue copper proteins includes those proteins in which links with five residues are coordinated: the azurins. The additional bond is formed by a carbonyl oxygen which binds to copper at the side opposite to methionine. The methionine residue is at a slightly larger distance than in plastocyanin (± 3.1 Å). The carbonyl group is at about 3.0 Å. The EPR spectrum of the proteins belonging to the first and second class are of the axial type and they only show one intense peak near 600 nm in the absorption spectrum.

A third class of blue copper proteins includes those proteins which have the same four coordinating links as in plastocyanin, but which are arranged in a more distorted conformation. The S_{Cys}-Cu(II) bond length increases simultaneously with the decrease of the S_{Met}-Cu(II) bond length. The EPR spectrum is rhombic and there is an increase of the absorption region of 460 nm. A fourth class of blue copper proteins is formed by those proteins that have an axial link with a residue that is different from methionine.

Although there is an appreciable amount of divergence in the sequence of the blue copper proteins, the copper ligand sites for many of the proteins are conserved and show the following pattern: [GA]- x(0,2) - [YSA]- x(0,1) - [VFY]- x-C-x(1,2) - [PG]- x(0,1)-H-x(2,4)- [MQ]. See http://Ibess.u-strasbg.fr/Biolnfo/Prosite/00174.pdoc. A special distorted trigonal planar arrangement of two histidine ligands and one cysteine ligand around the copper gives rise to the very peculiar electronic properties of the metal site: an intense blue color which is about 100 times more intense than most inorganic copper complexes (Cys-S-Cu charge transfer), axial EPR-spectra with small hyperfine coupling constants and high redox potentials. The apo metal binding protein for use in the practice of the invention is a blue copper protein with these characteristics.

The most well known members of this class of proteins are the plant chloroplastic plastocyanins, which exchange electrons with cytochrome c6, and the distantly related bacterial azurins, which exchange electrons with cytochrome 551. Other blue copper proteins include, but are not limited to, amicyanin from bacteria such as methylobacterium extorquens or thiobacillus versutus that can grow on methylamine; auracyanins A and B from chloroflexus aurantiacus; blue copper protein from alcaligenes faecalis; cupredoxin (CPC) from cucumber peelings; cusacyanin (basic blue protein, plantacyanin, CBP) from cucumber; halocyanin from natrobacterium pharaonis, a membrane associated copper-binding protein; pseudoazurin from pseudomonas; rusticyanin from thiobacillus ferrooxidans; stellacyanin from the Japanese lacquer tree; umecyanin from horseradish roots; and allergen Ra3 from ragweed. Garret T.P.J., et al., J. Biol. Chem. 259:2822-2825(1984); Ryden L.G., & Hunt L.T., J. Mol. Evol. 36:41-66(1993); McManus J.D., et al., J. Biol. Chem. 267:6531-6540(1992); Mann K., et al., FEBS Lett. 314:220-223(1992); Mattar S., et al., J. Biol. Chem. 269:14939-14945(1994); Yano T., et al., FEBS Lett. 288:159-162(1991).

The signal emitted by a blue copper protein may be any change or acquisition of color or change in the intensity of blue color. Color, as used herein, is defined as any chromatic signal. In one embodiment, the color observed or detected is in the visible or ultraviolet wavelength range. Color as defined herein may also be a fluorescent or luminescent signal. The signal may be observed or measured by any means known in the art including, but not limited to, visible detection by the naked eye, a microscope, photon tube, or diode. In one embodiment, the frequency, duration, intensity, and/or localization of the signal is measured or observed at a single point in time or as a function of time.

As defined herein, a biological system may be any *in* vivo or *in* vitro biological or biochemical system including but not limited to any cell or cell culture, virus, bacteria, plant, or animal or any part or simulated system thereof. In the practice of the invention, the target substance may be chosen from any cell, such as bacterial, fungal, plant or animal cell, any tissue, any virus, or any protein. Suitable animal sells include, but are not limited to Vero cells, HeLa cells, Cos cells, CV1 cells and various primary mammalian cells. In an embodiment, the bacterial cell is Escherichia coli. The target substance may also be a drug or therapeutic treatment that is labelled in order to follow its activity or function *in vivo* or *in vitro.* In one embodiment, the blue copper protein is linked, e.g., covalent or ionic bond, to a tissue, cell, and/or a protein, or a fusion protein is prepared between one or more proteins of interest and one or more blue copper proteins.

A cell may also be labelled by introducing a blue copper protein into the cell or by transfecting the cell with a DNA molecule encoding a blue copper protein. Blue copper proterin may also be bound or anchored to the membrane of a cell either inside or outside of the cell wall. The blue copper protein may be provided to a cell, tissue or protein of interest *in vivo* or *in vitro.*

Also within the practice of the invention is a eucaryotic cell comprising a DNA sequence encoding a blue copper protein. The eucaryotic cell may be an animal cell and in one embodiment, the DNA sequence comprises the azu gene.

In one embodiment the blue copper protein may be used as reporter gene. The reporter gene may be inserted into a cell and the cell may comprise a DNA molecule having a regulatory element from a gene, other than a gene encoding a blue copper protein operatively linked to a DNA sequence encoding the blue copper protein. As used herein, "a regulatory element" from a gene is the DNA sequence which is necessary for the expression of the gene. In this invention, the term "operatively linked" means that following such a link the regulatory element can direct the expression of the linked DNA sequence. A gene encoding a blue copper protein includes DNA molecules coding for polypeptide analogs, fragments or derivatives of antigenic polypeptides which differ from naturally-occurring forms in terms of the identity or location of one or more amino acid residues (deletion analogs containing less than all of the residues specified for the protein, substitution analogs wherein one or more residues specified are replaced by other residues and addition analogs where in one or more amino acid residues is added to a terminal or medial portion of the polypeptides) and which share some or all properties of naturally-occurring forms.

These DNA molecules include: the incorporation of codons "preferred" for expression by selected non-mammalian hosts; the provision of sites for cleavage by restriction endonuclease enzymes; and the provision of additional initial, terminal or intermediate DNA sequences that facilitate construction of readily expressed vectors.

In one embodiment, the blue copper proteins could be linked to a promotor that by itself is responsive to an intracellular process. Any influence (e.g. by a drug added to the cell) on that process would affect the expression rate of the metal binding protein and hence could be monitored by measuring the intensity of the color of the cell.

One of ordinary skill in the art will be able to determine the conditions which must be provided in order to permit a blue copper protein to emit a signal depending on the blue copper protein chosen and the application envisaged. The proper conditions may include, but are not limited to, varying pH, ionic strength, intracellular redox potential, providing a metal to bind to the blue copper protein, and altering the charge of the metal.

For example, to provide the conditions which permit azurin to emit a signal, *e.g.,* blue color, two conditions should be fulfilled: (1) azurin has to bind copper either before or after labeling the target substance, and (2) the protein has to be in an oxidized state. Furthermore, these conditions have to be compatible with the *in vivo* or culturing conditions of the biological system. Free Cu²⁺, for example, is toxic to the cells, even in relatively low concentrations.

Azurin binds copper with high affinity (the binding of copper is thermodynamically favored with the binding energy of Cu (II) exceeding those of other transition ions), and the resulting copper-protein complex is very stable. Therefore low, non-toxic, amounts of Cu²⁺ in the medium (in a range of 0,5 to 1 µM) should allow irreversible binding of the metal to azurin. In one embodiment, the copper-transport within a biological system may occur by trafficking proteins, which take the Cu²⁺ to different copper containing oxidases in the vesicular systems.

Not to be limited as to theory, the intense blue color of azurin is a property of the oxidized Cu(II) form, that possesses a strong visible absorption band at 628 nm (ε = 5700 M⁻¹cm⁻¹), while the Cu(I) form is colorless. The oxidized azurin is the most stable form, but the reduction potential of the Cu-complexes is strongly influenced by the medium and ionic strength, and the blue color of the Cu(II) complex fades with decreasing pH. Thus, in one embodiment, a change in medium or ionic strength of the medium may provide the condition that permits the blue copper protein to emit a signal.

In a further embodiment, the physiologic pH of the cell cytoplasm allows formation of the functional protein. However, since the reduction potential of the cell environment however is rather low, therefore favoring the colorless Cu(I) form, in one embodiment, a cell is lysed at the endpoint of the assay for detection of the blue color of the expressed azurin in a lysis buffer with compatible pH, copper concentration, and reduction potential.

In another embodiment, one may target the expressed blue copper protein to specific "oxidative" organelles like mitochondria, post-Golgi vesicles and peroxisomes. According to the biological role of azurin, for example, the mitochondria are the appropriate organelles for expression of the protein. Azurin belongs to a family of proteins which function in the respiratory chains of denitrifying bacteria and plants as electron-transfer agents. Their role is to transport electrons between different oxidases (e.g. cytochrome c and cytochrome oxidase, ATPases). The targeting of the proteins to the mitochondria can easily be accomplished by placing a mitochondrial targeting signal in front of the *azu* gene coding sequence.

Targeting of azurin to the mitochondria has an additional advantage on the concentration of the expressed protein. Although expression of the pLNCX-BCP1/2 constructs is induced by the strong constitutive CMV-promoter, the amount of azurin in some systems may be insufficient for efficient detection of the blue color, because there is no enzymatic amplification of the signal (= blue color). Targeting the proteins to the mitochondria concentrates the blue color which should allow a more accurate detection. The protein yield can also be enhanced by placing a translational enhancer directly upstream of the coding sequence of azurin. In that way up to five-fold higher levels of expression can be achieved. As a result of targeting and enhanced expression, the mitochondrial blue "pixels" should be easily detected and measured with, for example, an electron microscope, a confocal microscope, a color camera or the Tibotec multisampling microscope as described in US Patent publication No US2003/0142398.

There are countless applications in which the methods and compositions of the present invention may be applied. For example, in one embodiment, the invention provides for a method for detecting at least one cell expressing a protein of interest. A DNA molecule having a DNA sequence encoding one or more protein(s) of interest and an additional DNA molecule having a DNA sequence encoding one or more blue copper protein(s) may be inserted into the cell. By providing conditions which permit the blue copper protein to emit a signal, one may detect the cell expressing the protein of interest by observing or measuring the signal of the blue copper protein. The DNA molecule and the additional DNA molecule may be linked.

In another embodiment, the invention provides a method for localizing at least one protein of interest in a cell. Here, introduced into the cell is a DNA molecule comprising a DNA sequence encoding the at least one protein of interest, linked to an additional DNA sequence encoding a blue copper protein. The sequences are linked such that the protein produced by the DNA molecule will have the at least one protein of interest fused to the blue copper protein. The location of the fused protein may be determined from the signal of the expressed fusion protein, thereby localizing the protein of interest in the cell.

The invention includes the use of these proteins or peptides in drug discovery technologies and diagnostic testing. In one embodiment, the invention provides for a drug or a treatment discovered, identified or designed using the methods described herein.

The methods of the invention are especially applicable in high throughput testing or evaluation devices. The invention could also be used in high throughput screening for drugs. In this area the speed of the screening is considered as one of the most important bottlenecks in the process of drug discovery. A simplification of the detection of the reporter or tag is one way to speed up the screening process. The blue copper proteins are ideal for this purpose. There would be no need for extra manipulations for detection and thus could be used in homogenous assays, making them amenable to automation. The fact that there's no need for sophisticated and expensive detection equipment is another important direct advantage

It is within the practice of the invention to prepare a sample rack or solid support made up of numerous reaction wells, such that each reaction remains isolated form one another. Simultaneous transfer of one or more reagents to the reaction wells may then be achieved by one of the many techniques used in the art of high throughput analysis.

One or more of the contents of each of the reaction wells may be varied. For example, in one embodiment, each reaction well contains the same biological system and target substance labeled with a blue copper protein. However, a drug is then added to each reaction well. The reaction wells may form an array or may employ another means of identifying or addressing each compartment. The activity of each reaction well may then be automatically determined from the amount of signal from a blue copper protein detected, and recorded. Other embodiments include, but are not limited to varying the concentration of one or more of the components.

There are numerous methods for handling high throughput. *i.e.,* analyzing a large number of samples in a relatively short period of time. Any method of high throughput analysis available may be applied to the methods of the invention. Examples include, but are not limited to: U.S. Patent No. 5,985,215 of Sakazume et al., entitled Analyzing Apparatus Having a Function Pipette Samples; U.S. Patent No. 6,046,056 of Parce et al., entitled High Throughput Screening Assay Systems in Microscale Fluidic Devices; WO 00/14540 of Pauwels et al., entitled Method For the Rapid Screening of Analytes; WO 99/30154 of Beutel et al., entitled Continuous Format High Throughput Screening; and WO 99/67639 of Wada et al., entitled High Throughput Methods, Systems and Apparatus for Performing Cell Based Screening Assays,

In a specific embodiment, blue copper proteins may be used as reporter in diagnostic testing, such as drug sensitivity analysis, as performed according to WO97/27480. In one embodiment, the invention provides a method of screening a therapeutic agent for treating a disease comprising labeling at least one cell that is a target of the disease with a blue copper protein. Whether the therapeutic agent is effective treating the disease is determined by monitoring the signal observed or measured from the blue copper protein and thus the cell.

The invention also provides a method of screening a therapeutic agent for treating a virus or bacterial infection. The virus or the bacteria is labeled with a blue copper protein and conditions are provided which permit the blue copper protein to emit a signal. Whether the therapeutic agent is effective treating the virus or bacterial infection is determined by monitoring the signal observed or measured from the virus.

In one embodiment, the virus is the HIV virus and the blue copper proteins are used for the screening of drugs that are effective against HIV. For example, the gene coding for this protein could be operatively linked to a constitutively active promotor of a CD4⁺ cell. If the drug is inactive against the virus, the addition of the drug and the HIV virus to a CD4⁺ cell will kill that cell because the virus is able to enter and kill the cell. The cell will express the metal binding protein no more and will loose its color. If it is a drug active against HIV, the virus can not kill the cell and the cell will keep its color.

The cytotoxicity of drugs could also be determined by the use of these novel markers. In this case, the gene coding for the blue copper protein could be operatively linked to a constitutively active promotor and introduced in a cell. Living cells, which are not influenced by a drug would be detected as they continue to support gene expression and thus express the metal binding protein giving the cell an intense color. If a drug is toxic to the cell, there will be no more expression of the marker and the color of the cell would disappear.

In addition one could envision the use of blue copper protein or related peptide in almost any application in which a green fluorescent protein (GFP) (or related mutants thereof) is used. Many uses of GFP are disclosed/covered by US 5,491,084. One example is the use of (modified) GFP in a farnesyl-transferase assay we described recently (PCT/EP00/04923 and PCT/EP/004919) in which a colored metal binding protein would be incorporated in a fusion protein containing a cleavable site (such as DVED for caspases) together with Ras sequences that target the entire fusion protein to the plasma membrane. Any action of a protease, such as caspase, would cause a detectable redistribution of the metal binding protein within the cell.

A blue copper protein may also replace currently used reporters in diagnostic testing such as the anti Virogram™ procedure as disclosed in WO97/27480.

The invention also provides for a kit. The kit may be used for any of the methods described herein. In one embodiment, the kit may be used to monitor at least one target substance in a biological system. The kit of the invention may comprise a blue copper protein and/or a DNA sequence encoding a blue copper protein. The kit may further comprise a plasmid for delivering the DNA sequence encoding a blue copper protein to a cell. In another embodiment, a kit comprises a eucaryotic cell comprising a DNA sequence encoding a blue copper protein.

### Examples

### Example 1: Construction of pLNCX-BCP1 and pLNCX-BCP2

The bacteria *Achromobacter xylosoxidans* subsp. *denitrificans* (or *Alcaligenes denitrificans)* was purchased from the ATCC (ATCC number 15173, NCTC 8582). The gene *azu* coding for azurin was PCR-amplified from bacterial DNA and cloned into a mammalian expression vector. The *azu* gene codes for a pre-protein in which the mature azurin (130 AA) is preceded by a 19-AA signal peptide. This peptide probably serves the purpose of translocating the protein across the periplasmic membrane. The complete protein (with signal peptide) was cloned in order not to have difficulties with correct protein folding. In a second construct a secretion signal (= Ig κ-leader sequence) was placed before the gene, to allow secretion into the medium.

The *azu* gene was cloned into the retroviral vector pLNCX (Clontech). This vector, derived from Moloney murine leukemia virus (MoMuLV), is designed for retroviral gene delivery and expression. It contains a neomycin resistance (Neo^{r}) gene controlled by 5' viral LTR (5' LTR) for antibiotic selection in eukaryotic cells and a cloning site *(Hind* III, *Hpa*I and *Cla*I) downstream from a cytomegalovirus (Pcmv) immediate early promotor. The Ψ⁺sequence is an extended viral packaging signal required for the viral vector transcript to be packaged in virions. pLNCX does not contain the viral structural genes (gag-pol and env) necessary for particle formation and replication, however, they can be provided in trans in packaging cell lines stably expressing these genes.

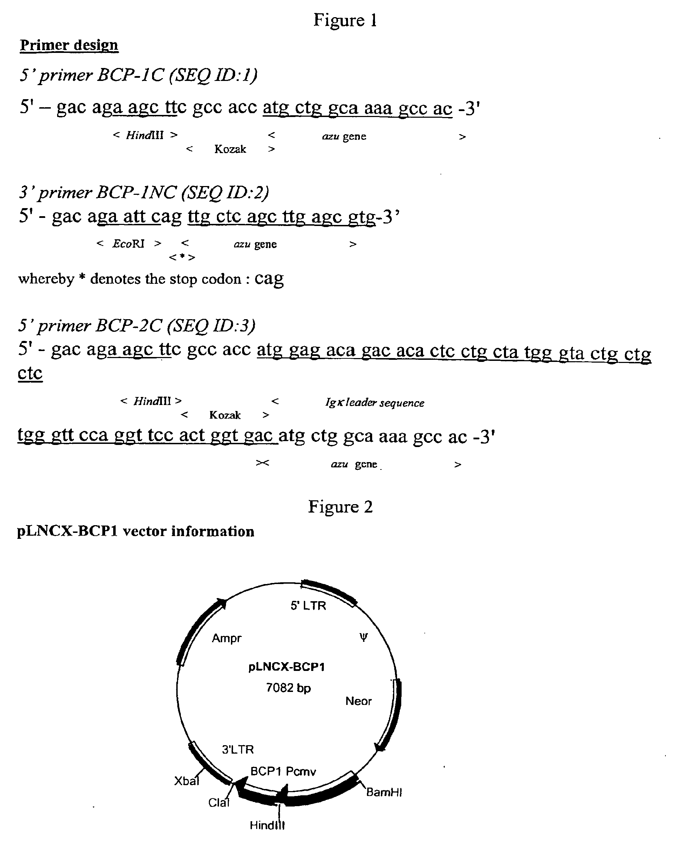

The *azu* gene was amplified from bacterial DNA by PCR using the 5' primers BCP-1C (SEQ ID:1) or BCP-2C (SEQ ID:3), and the 3' primer BCP-1NC (SEQ ID:2) (Figure 1). The 5' primers are designed containing a translation initiation (Kozak) sequence before the initiating ATG codon. The primer BCP-2C (SEQ ID:3) includes the Ig κ-leader sequence for secretion of BCP into the medium. The 3' primers include the native stop codon of the protein. The primers also contain a restriction site *(Hind*III for the 5' primers and EcoRI for the 3' primers) that allows an easy subcloning of the *azu* gene.

The PCR product was, after restriction with *Hind*III*,* cloned into the *Hind*III*-Hpa*I site of pLNCX (Figure 2).

### Example 2: Transfection and detection of azurin in eukaryotic cells

The protein azurin is a blue copper protein that is characterized by an intense absorption near 600 nM causing a blue color when Cu²⁺ is bound to the protein. The protein is expressed by different strains of bacteria. The blue colored protein was expressed in eukaryotic cells, and used as an alternative for different reporter systems (β-gal, GFP, ... ) in cell-based assays. The experiment showed that constitutive expression and detection of the protein could be used as a viability marker.

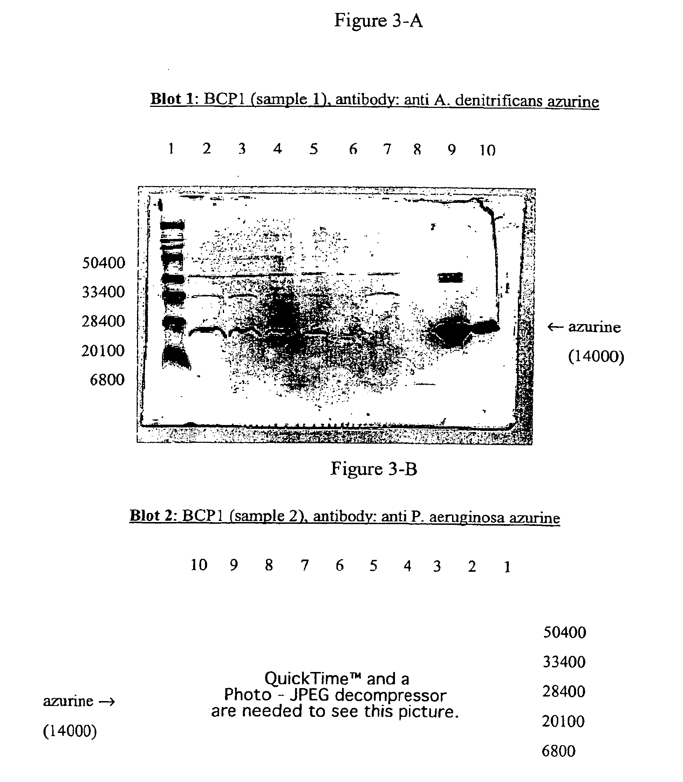

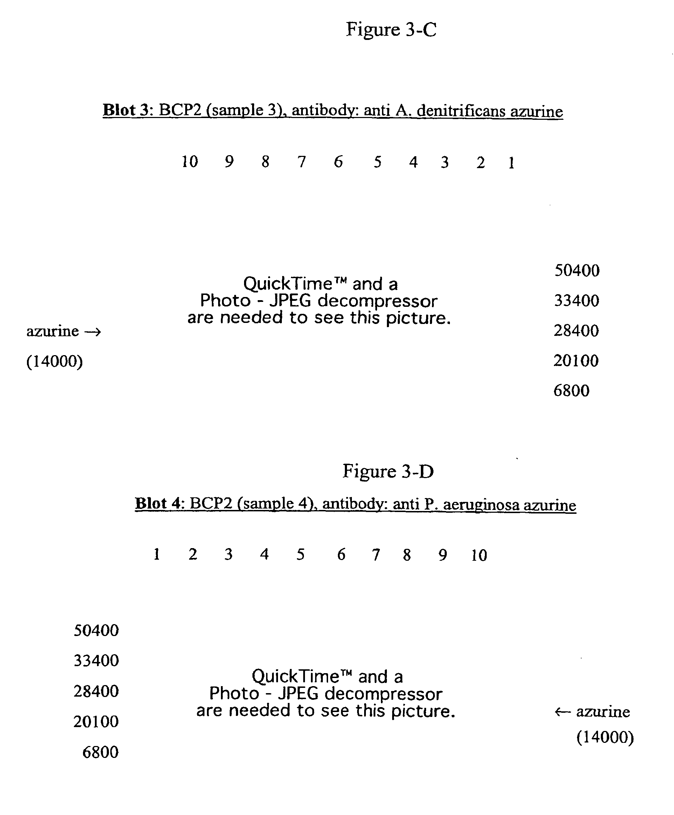

293T cells, T-antigen transformed human embryonic kidney cells, were transiently transfected with the constructs pLNXC-BCP 1 or pLNCX-BCP2 using Effectene transfection reagent (Qiagen). SDS-PAGE (15%) followed by Western blotting of the cell lysates confirmed expression of azurin by both constructs (Figure 3).

The azurin was detected in cell lysates (Cell lysates (10⁶ cells/sample)) with polyclonal antibody anti A. denitrificans azurin (Leiden Institute of chemistry, Gorlaeus laboratories). The Western blots shown in Figure 3 may be interpreted as follows:

| | |
|---|---|
| Lane 1: Biorad Prestained Marker Broadrange | Lane 2: 15 µl sample |
| Lane 3: 10 µl sample | Lane 4: 7.5 µl sample |
| Lane 5: 5 µl sample | Lane 6: 2.5 µl sample |
| Lane 7: negative control (non-transfected cells) | Lane 8: empty |
| Lane 9: A. denitrificans azurine | Lane 10; P. aeruginosa azurine |

## Claims

1. A method of monitoring at least one target substance in an *in vitro* biological system comprising:
a) providing said biological system comprising said at least one target substance;
b) labeling said at least one target substance with at least one blue copper protein;
c) providing conditions which permit said at least one blue copper protein to emit a signal;
d) observing or measuring the signal;
e) monitoring said at least one target substance based on the signal observed or measured.

2. The method of claim 1, wherein said step of monitoring comprises determining the location of said at least one target substance in said biological system.

3. The method of claim 1, wherein said step of monitoring comprises quantifying the amount of said at least one target substance in said biological system.

4. The method of claim 1, wherein said biological system is chosen from a virus, bacteria, plant, or animal.

5. The method of claim 1, wherein said at least one target substance is chosen from a cell or a tissue.

6. The method of claim 5, wherein said cell is chosen from a bacterial, fungal, plant or animal cell.

7. The method of claim 5, wherein said cell is labelled by transfecting said cell with a DNA molecule encoding said at least one blue copper protein.

8. The method of claim 5, wherein said cell is labeled by linking said at least one blue copper protein to said cell.

9. The method of claim 5, wherein said at least one blue copper protein is provided to said cell *in vivo.*

10. The method of claim 5, wherein said at least one blue copper protein is provided to said cell *in vitro.*

11. A method of determining the cytotoxicity of a drug of interest comprising
a) exposing at least one cell to the drug of interest;
b) monitoring the at least one cell using the method of claim 5;
c) determine the cytotoxicity of the drug of interest by determining whether the at least one cell is influenced by the drug of interest.

12. The method of claim 5, wherein said at least one blue copper protein is linked to said tissue.

13. The method of claim 1, wherein said at least one target substance is a protein.

14. The method of claim 13, wherein said protein is labelled by preparing a fusion protein with said protein and at least one blue copper protein.

15. The method of claim 1, wherein said conditions which permit said at least one blue copper protein to emit a signal comprise providing copper which binds to said at least one blue copper protein.

16. The method of claim 15, wherein said blue copper protein is chosen from azurin, pseudo-azurin, a plastocyanin, and a phytocyanin.

17. The method of claim 15, wherein said blue copper protein is a blue oxidase chosen from ascorbate oxidase, ceruloplasmin, and laccase.

18. The method of claim 15, wherein said blue copper protein is a nitrite reductase.

19. A method for detecting at least one cell expressing a protein of interest comprising
a) introducing into said at least one cell a DNA molecule having a DNA sequence encoding the protein of interest and an additional DNA molecule having a DNA sequence encoding at least one blue copper protein;
b) providing conditions which permit expression of the at least one blue copper protein and the protein of interest;
c) providing conditions which permit the at least one blue copper protein to emit a signal; and
d) detecting the cell expressing the protein of interest by observing or measuring the signal of the at least one blue copper protein.

20. The method of claim 19, wherein said DNA molecule and said additional DNA molecule are linked.

21. The method of claim 19, wherein said cell is chosen from bacterial, fungal, plant or animal cell.

22. A method for localizing at least one protein of interest in a cell comprising
a) introducing into said cell a DNA molecule comprising a DNA sequence encoding the at least one protein of interest, linked to an additional DNA sequence encoding at least one blue copper protein such that the protein produced by the DNA molecule will have the at least one protein of interest fused to the at least one blue copper protein;
b) providing conditions which permit expression of the fused protein;
c) providing conditions which permit the at least one blue copper protein to emit a signal; and
d) determining the location of the fused protein from the signal, thereby localizing the protein of interest in the cell.

23. A method of screening a therapeutic agent for treating a disease comprising:
a) labeling at least one cell that is a target of the disease with at least one blue copper protein;
b) providing conditions which permit said at least one blue copper protein to emit a signal;
c) observing or measuring the signal;
d) determining whether the therapeutic agent is effective treating the disease by monitoring the signal observed or measured from the cell.

24. A method of screening a therapeutic agent for treating a virus or bacterial infection comprising:
a) labeling the virus or the bacteria with at least one blue copper protein;
b) providing conditions which permit said at least one blue copper protein to emit a signal;
c) observing or measuring the signal;
d) determining whether the therapeutic agent is effective treating the virus or bacterial infection by monitoring the signal observed or measured from the virus or the bacteria.

25. The method according to claim 24, wherein said virus is the HIV virus.

26. A eucaryotic cell comprising a DNA sequence encoding azurin.

27. The eucaryotic cell of claim 26, wherein said eucaryotic cell is an animal cell.

28. The eucaryotic cell of claim 26, wherein said DNA sequence comprises the azu gene.

## Patentansprüche

1. Verfahren zum Überwachen mindestens einer Zielsubstanz in einem biologischen *In-vitro-*System, umfassend:
a) Bereitstellen des biologischen Systems, das die mindestens eine Zielsubstanz umfasst,
b) Markieren der mindestens einen Zielsubstanz mit mindestens einem blauen Kupferprotein,
c) Bereitstellen von Bedingungen, die es dem mindestens einen blauen Kupferprotein gestatten, ein Signal auszusenden,
d) Beobachten oder Messen des Signals,
e) Überwachen der mindestens einen Zielsubstanz auf Basis des beobachteten oder gemessenen Signals.

2. Verfahren nach Anspruch 1, wobei der Schritt des Überwachens das Bestimmen des Ortes der mindestens einen Zielsubstanz in dem biologischen System umfasst.

3. Verfahren nach Anspruch 1, wobei der Schritt des Überwachens das Quantifizieren der Menge der mindestens einen Zielsubstanz in dem biologischen System umfasst.

4. Verfahren nach Anspruch 1, wobei das biologische System aus einem Virus, Bakterien, einer Pflanze oder einem Tier ausgewählt ist.

5. Verfahren nach Anspruch 1, wobei die mindestens eine Zielsubstanz aus einer Zelle oder einem Gewebe ausgewählt ist.

6. Verfahren nach Anspruch 5, wobei die Zelle aus einer Bakterien-, Pilz-, Pflanzen- oder Tierzelle ausgewählt ist.

7. Verfahren nach Anspruch 5, wobei die Zelle durch Transfizieren der Zelle mit einem DNA-Molekül, welches das mindestens eine blaue Kupferprotein codiert, markiert wird.

8. Verfahren nach Anspruch 5, wobei die Zelle durch Verknüpfen des mindestens einen blauen Kupferproteins mit der Zelle markiert wird.

9. Verfahren nach Anspruch 5, wobei das mindestens eine blaue Kupferprotein der Zelle *in vivo* zur Verfügung gestellt wird.

10. Verfahren nach Anspruch 5, wobei das mindestens eine blaue Kupferprotein der Zelle *in vitro* zur Verfügung gestellt wird.

11. Verfahren zur Bestimmung der Cytotoxizität eines Medikaments, umfassend
a) Aussetzen mindestens einer Zelle dem Medikament von Interesse,
b) Überwachen der mindestens einen Zelle unter Verwendung des Verfahrens nach Anspruch 5,
c) Bestimmen der Cytotoxizität des Medikaments von Interesse durch Bestimmen, ob die mindestens eine Zelle durch das Medikament von Interesse beeinflusst wird.

12. Verfahren nach Anspruch 5, wobei das mindestens eine blaue Kupferprotein mit dem Gewebe verknüpft wird.

13. Verfahren nach Anspruch 1, wobei die mindestens eine Zielsubstanz ein Protein ist.

14. Verfahren nach Anspruch 13, wobei das Protein durch Herstellen eines Fusionsproteins mit dem Protein und mindestens einem blauen Kupferprotein markiert wird.

15. Verfahren nach Anspruch 1, wobei die Bedingungen, die es dem mindestens einen blauen Kupferprotein gestatten, ein Signal auszusenden, das Bereitstellen von Kupfer umfassen, das an das mindestens eine blaue Kupferprotein bindet.

16. Verfahren nach Anspruch 15, wobei das blaue Kupferprotein aus Azurin, Pseudoazurin, einem Plastocyanin und einem Phytocyanin ausgewählt ist.

17. Verfahren nach Anspruch 15, wobei es sich bei dem blauen Kupferprotein um eine aus Ascorbatoxidase, Ceruloplasmin und Laccase ausgewählte blaue Oxidase handelt.

18. Verfahren nach Anspruch 15, wobei es sich bei dem blauen Kupferprotein um eine Nitritreduktase handelt.

19. Verfahren zum Nachweisen mindestens einer Zelle, die ein Protein von Interesse exprimiert, umfassend:
a) Einbringen eines DNA-Moleküls mit einer DNA-Sequenz, die das Protein von Interesse codiert, und eines zusätzlichen DNA-Moleküls mit einer DNA-Sequenz, die mindestens ein blaues Kupferprotein codiert, in die mindestens eine Zelle,
b) Bereitstellen von Bedingungen, welche die Expression des mindestens einen blauen Kupferproteins und des Proteins von Interesse gestatten,
c) Bereitstellen von Bedingungen, die es dem mindestens einen blauen Kupferprotein gestatten, ein Signal auszusenden, und
d) Nachweisen der Zelle, die das Protein von Interesse exprimiert, durch Beobachten oder Messen des Signals von dem mindestens einen blauen Kupferprotein.

20. Verfahren nach Anspruch 19, wobei das DNA-Molekül und das zusätzliche DNA-Molekül verknüpft sind.

21. Verfahren nach Anspruch 19, wobei die Zelle aus einer Bakterien-, Pilz-, Pflanzen- oder Tierzelle ausgewählt ist.

22. Verfahren zum Lokalisieren mindestens eines Proteins von Interesse in einer Zelle, umfassend:
a) Einbringen eines DNA-Moleküls, das eine DNA-Sequenz, die das mindestens eine Protein von Interesse codiert, verknüpft mit einer zusätzlichen DNA-Sequenz umfasst, die mindestens ein blaues Kupferprotein codiert, in die Zelle, so dass bei dem von dem DNA-Molekül hergestellten Protein das mindestens eine Protein von Interesse an das mindestens eine blaue Kupferprotein fusioniert ist,
b) Bereitstellen von Bedingungen, welche die Expression des Fusionsproteins gestatten,
c) Bereitstellen von Bedingungen, die es dem mindestens einen blauen Kupferprotein gestatten, ein Signal auszusenden, und
d) Nachweisen des Ortes des Fusionsproteins anhand des Signals, wodurch das Protein von Interesse in der Zelle lokalisiert wird.

23. Verfahren zum Screenen eines Therapeutikums zur Behandlung einer Erkrankung, umfassend:
a) Markieren mindestens einer Zelle, die ein Ziel der Erkrankung ist, mit mindestens einem blauen Kupferprotein,
b) Bereitstellen von Bedingungen, die es dem mindestens einen blauen Kupferprotein gestatten, ein Signal auszusenden,
c) Beobachten oder Messen des Signals,
d) Bestimmen, ob das Therapeutikum zur Behandlung der Erkrankung wirksam ist, durch Überwachen des beobachteten oder gemessenen Signals von der Zelle.

24. Verfahren zum Screenen eines Therapeutikums zur Behandlung einer Virus- oder Bakterieninfektion, umfassend:
a) Markieren des Virus oder der Bakterien mit mindestens einem blauen Kupferprotein,
b) Bereitstellen von Bedingungen, die es dem mindestens einen blauen Kupferprotein gestatten, ein Signal auszusenden,
c) Beobachten oder Messen des Signals,
d) Bestimmen, ob das Therapeutikum zur Behandlung der Virus- oder Bakterieninfektion wirksam ist, durch Überwachen des beobachteten oder gemessenen Signals von dem Virus oder den Bakterien.

25. Verfahren nach Anspruch 24, wobei es sich bei dem Virus um das HIV-Virus handelt.

26. Eukaryotische Zelle, die eine Azurin codierende DNA-Sequenz umfasst.

27. Eukaryotische Zelle nach Anspruch 26, wobei die eukaryotische Zelle eine Tierzelle ist.

28. Eukaryotische Zelle nach Anspruch 26, wobei die DNA-Sequenz das azu-Gen umfasst.

## Revendications

1. Procédé de suivi d'au moins une substance cible dans un système biologique *in vitro* comprenant :
a) une obtention dudit système biologique comprenant ladite au moins une substance cible,
b) un marquage de ladite au moins une substance cible avec au moins une protéine à cuivre bleu,
c) une obtention de conditions qui permettent à ladite au moins une protéine à cuivre bleu d'émettre un signal,
d) une observation ou mesure du signal,
e) un suivi de ladite au moins une substance cible basé sur le signal observé ou mesuré.

2. Procédé selon la revendication 1, dans lequel ladite étape de suivi comprend la localisation de ladite au moins une substance cible dans ledit système biologique.

3. Procédé selon la revendication 1, dans lequel ladite étape de suivi comprend une quantification de la quantité de ladite au moins une substance cible dans ledit système biologique.

4. Procédé selon la revendication 1, dans lequel ledit système biologique est choisi parmi un virus, une bactérie, un système végétal ou animal.

5. Procédé selon la revendication 1, dans lequel ladite au moins une substance cible est choisie parmi une cellule ou un tissu.

6. Procédé selon la revendication 5, dans lequel ladite cellule est choisie parmi une cellule bactérienne, fongique, végétale ou animale.

7. Procédé selon la revendication 5, dans lequel ladite cellule est marquée par une transfection de ladite cellule avec une molécule d'ADN codant pour ladite au moins une protéine à cuivre bleu.

8. Procédé selon la revendication 5, dans lequel ladite cellule est marquée par un attachement de ladite au moins une protéine à cuivre bleu à ladite cellule.

9. Procédé selon la revendication 5, dans lequel ladite au moins une protéine à cuivre bleu est procurée à ladite cellule *in vivo.*

10. Procédé selon la revendication 5, dans lequel ladite au moins une protéine à cuivre bleu est procurée à ladite cellule *in vitro.*

11. Procédé de détermination de la cytotoxicité d'un médicament d'intérêt comprenant :
a) une exposition d'au moins une cellule au médicament d'intérêt,
b) un suivi de la au moins une cellule en utilisant le procédé de la revendication 5,
c) une détermination de la cytotoxicité du médicament d'intérêt en déterminant si la au moins une cellule est influencée par le médicament d'intérêt.

12. Procédé selon la revendication 5, dans lequel ladite au moins une protéine à cuivre bleu est liée audit tissu.

13. Procédé selon la revendication 1, dans lequel ladite au moins une substance cible est une protéine.

14. Procédé selon la revendication 13, dans lequel ladite protéine est marquée en préparant une protéine fusion avec ladite protéine et au moins une protéine à cuivre bleu.

15. Procédé selon la revendication 1, dans lequel lesdites conditions qui permettent à ladite au moins une protéine à cuivre bleu d'émettre un signal comprennent la procuration du cuivre qui se lie à ladite au moins une protéine à cuivre bleu.

16. Procédé selon la revendication 15, dans lequel ladite protéine à cuivre bleu est choisie parmi de l'azurine, de la pseudoazurine, une plastocyanine et une phytocyanine.

17. Procédé selon la revendication 15, dans lequel ladite protéine à cuivre bleu est une oxydase bleue choisie parmi une oxydase d'ascorbate, une céruloplasmine et une laccase.

18. Procédé selon la revendication 15, dans lequel ladite protéine à cuivre bleu est une réductase de nitrite.

19. Procédé de détection d'au moins une cellule exprimant une protéine d'intérêt comprenant :
a) une introduction dans ladite au moins une cellule d'une molécule d'ADN comportant une séquence d'ADN codant pour la protéine d'intérêt et une molécule d'ADN additionnelle comportant une séquence d'ADN codant pour au moins une protéine à cuivre bleu,
b) une obtention de conditions qui permettent l'expression de la au moins une protéine à cuivre bleu et de la protéine d'intérêt,
c) une obtention de conditions qui permettent à la au moins une protéine à cuivre bleu d'émettre un signal, et
d) une détection de la cellule exprimant la protéine d'intérêt en observant ou en mesurant le signal de la au moins une protéine à cuivre bleu.

20. Procédé selon la revendication 19, dans lequel ladite molécule d'ADN et ladite molécule d'ADN additionnelle sont liées.

21. Procédé selon la revendication 19, dans lequel ladite cellule est choisie parmi une cellule bactérienne, fongique, végétale ou animale.

22. Procédé de localisation d'au moins une protéine d'intérêt dans une cellule comprenant :
a) une introduction dans ladite cellule d'une molécule d'ADN comportant une séquence d'ADN codant pour la au moins une protéine d'intérêt, liée à une séquence d'ADN additionnelle codant pour au moins une protéine à cuivre bleu de sorte que la protéine produite par la molécule d'ADN comportera la au moins une protéine d'intérêt fusionnée à la au moins une protéine à cuivre bleu,
b) une obtention de conditions qui permettent l'expression de la protéine fusionnée,
c) une obtention de conditions qui permettent à la au moins une protéine à cuivre bleu d'émettre un signal, et
d) une localisation de la protéine fusionnée à partir du signal, pour localiser de cette manière la protéine d'intérêt dans la cellule.

23. Procédé de criblage d'un agent thérapeutique pour le traitement d'une maladie comprenant :
a) un marquage d'au moins une cellule qui est une cible de la maladie avec au moins une protéine à cuivre bleu,
b) une obtention de conditions qui permettent à ladite au moins une protéine à cuivre bleu d'émettre un signal,
c) une observation ou mesure du signal,
d) une détermination du fait que l'agent thérapeutique est efficace ou non pour le traitement de la maladie en suivant le signal observé ou mesuré à partir de la cellule.

24. Procédé de criblage d'un agent thérapeutique pour le traitement d'une infection virale ou bactérienne comprenant :
a) un marquage du virus ou de la bactérie avec au moins une protéine à cuivre bleu,
b) une obtention de conditions qui permettent à ladite au moins une protéine à cuivre bleu d'émettre un signal,
c) une observation ou mesure du signal,
d) une détermination du fait que l'agent thérapeutique est efficace ou non pour le traitement de l'infection virale ou bactérienne en suivant le signal observé ou mesuré à partir du virus ou de la bactérie.

25. Procédé selon la revendication 24, dans lequel ledit virus est le virus VIH.

26. Cellule eucaryote comprenant une séquence d'ADN codant pour l'azurine.

27. Cellule eucaryote selon la revendication 26, dans laquelle ladite cellule eucaryote est une cellule animale.

28. Cellule eucaryote selon la revendication 26, dans laquelle ladite séquence d'ADN comprend le gène *azu.*
